# EUROPEAN PATENT APPLICATION

(11) **EP 3 909 625 A1**
(43) Date of publication of application: **17.11.2021**
(21) Application number: 20745523.9
(22) Date of filing: 09.01.2020
(51) Int. Cl.: A61M 5/28, A61M 5/31

(54) **SYRINGE CAP ASSEMBLY**

(30) Priority: 21.01.2019 JP 2019007497
(71) Applicant: Nipro Corporation, Osaka-shi, Osaka 531-8510 (JP)
(72) Inventor: IGUCHI, Yuya, Osaka-shi, Osaka 531-8510 (JP); KANEMURA, Koichi, Osaka-shi, Osaka 531-8510 (JP)
(74) Representative: Prüfer & Partner mbB Patentanwälte · Rechtsanwälte
(86) International application number: PCT/JP2020/000383
(87) International publication number: WO 2020/153131

(57) **Abstract**

A syringe cap assembly (10) to be attached to an ejection port at a proximal end of a syringe (11) includes: a lock collar (13) to be attached to the ejection port at the proximal end of the syringe (11); and a plastic cap (14) screwed and thereby tightened into the lock collar (13). An external surface of the lock collar (13) has a plurality of projecting ribs (13a) that are each extended in an axial direction and are arranged in a circumferential direction to define a plurality of recessed grooves (13b) that are each extended in the axial direction and located between adjacent projecting ribs (13a) of the plurality of projecting ribs (13a) that are adjacent to each other in the circumferential direction. A bottom of an external surface of the plastic cap (14) includes a protrusion (14p) extending in a direction toward the lock collar (13). At a stage where tightening rotation of the plastic cap (14) implemented by screwing the plastic cap (14) into the lock collar (13) is completed, the protrusion (14p) is located in the recessed groove (13b) between the adjacent projecting ribs (13a).

## Description

### TECHNICAL FIELD

The present invention relates to a syringe cap assembly.

### BACKGROUND ART

A prefilled syringe filled aseptically with a liquid medicine has been known. For example, Japanese Patent Laying-Open No. 2007-029561 (PTL 1) discloses a technique relating to this prefilled syringe.

### CITATION LIST

### PATENT LITERATURE

PTL 1: Japanese Patent Laying-Open No. 2007-029561

### SUMMARY OF INVENTION

### TECHNICAL PROBLEM

A prefilled syringe has a syringe cap assembly attached to an ejection port at the proximal end of a syringe. Specifically, the syringe cap assembly includes a lock collar and a plastic cap. The lock collar is attached directly to the ejection port at the proximal end of the syringe. The plastic cap is tightened by being screwed into the lock collar and accordingly fastened to the lock collar. A rubber cap is inserted in the plastic cap, and the rubber cap abuts against and covers the ejection port at the proximal end of the syringe to thereby ensure the liquid tightness of the ejection port at the proximal end of the syringe.

The prefilled syringe filled with a liquid medicine is transported in an individual package. Vibrations or the like occurring during the transportation may cause micro-vibrations of the plastic cap, which may cause the plastic cap tightened into the lock collar to be loosened. In this case, the aseptic condition of the liquid medicine is endangered.

The present invention has been made in view of the above problem, and an object of the present invention is to provide a syringe cap assembly configured to enable prevention of loosening of a plastic cap tightened into a lock collar.

### SOLUTION TO PROBLEM

The syringe cap assembly is a syringe cap assembly to be attached to an ejection port at a proximal end of a syringe. The syringe cap assembly includes: a lock collar to be attached to the ejection port at the proximal end of the syringe; and a plastic cap screwed and thereby tightened into the lock collar. An external surface of the lock collar has a plurality of projecting ribs that are each extended in an axial direction and are arranged in a circumferential direction to define a plurality of recessed grooves, each recessed groove of the plurality of recessed grooves is extended in the axial direction and located between adjacent projecting ribs of the plurality of projecting ribs, and the adjacent projecting ribs are adjacent to each other in the circumferential direction. A bottom of an external surface of the plastic cap includes a protrusion extending in a direction toward the lock collar. At a stage where tightening rotation of the plastic cap implemented by screwing the plastic cap into the lock collar is completed, the protrusion is located in the recessed groove between the adjacent projecting ribs.

According to an another aspect, the plurality of projecting ribs are arranged at regular intervals on the external surface of the lock collar to allow the plurality of recessed grooves to be defined at regular intervals on the external surface of the lock collar. At the stage where tightening rotation of the plastic cap implemented by screwing the plastic cap into the lock collar is completed, the protrusion is located near one of the projecting ribs that is located upstream in a rotational direction of the plastic cap.

According to another aspect, the plurality of projecting ribs are arranged at eight respective locations at regular intervals on the external surface of the lock collar.

According to another aspect, the protrusion is placed at each of total two locations facing each other with an angle of 180 degrees between the two locations.

### ADVANTAGEOUS EFFECTS OF INVENTION

The syringe cap assembly makes it possible to provide a syringe cap assembly configured to enable prevention of loosening of the plastic cap tightened into the lock collar.

### BRIEF DESCRIPTION OF DRAWINGS

Fig. 1 shows an overall configuration of a prefilled syringe according to an embodiment.
Fig. 2 is a perspective view showing a syringe cap assembly to be attached to the proximal end of a syringe according to an embodiment.
Fig. 3 is a perspective view as seen from the bottom of a plastic cap according to an embodiment.
Fig. 4 is a cross-sectional view as seen in the direction of an arrow-headed line IV-IV in Fig. 2.
Fig. 5 is a perspective view as seen in the direction of an arrow-headed line V-V in Fig. 4.

### DESCRIPTION OF EMBODIMENTS

A prefilled syringe according to an embodiment is described hereinafter with reference to the drawings. The number, amount, or the like mentioned in connection with embodiments described below does not necessarily limit the scope of the present invention to the mentioned number, amount, or the like, unless otherwise specified. The same parts or corresponding parts are denoted by the same reference numerals, and a description thereof may not be repeated herein. It is intended originally that features of the embodiments may be combined and used appropriately. The drawings are not necessarily drawn to actual scale, and some drawings are drawn to a different scale for facilitating understanding of the structure.

### Prefilled Syringe 1

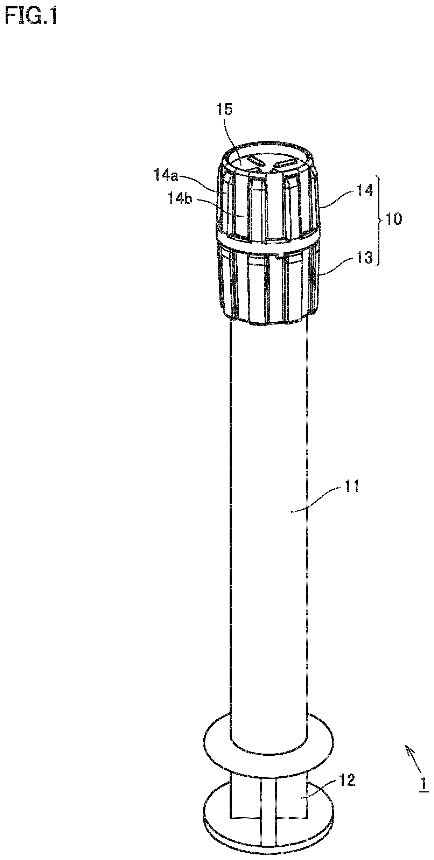

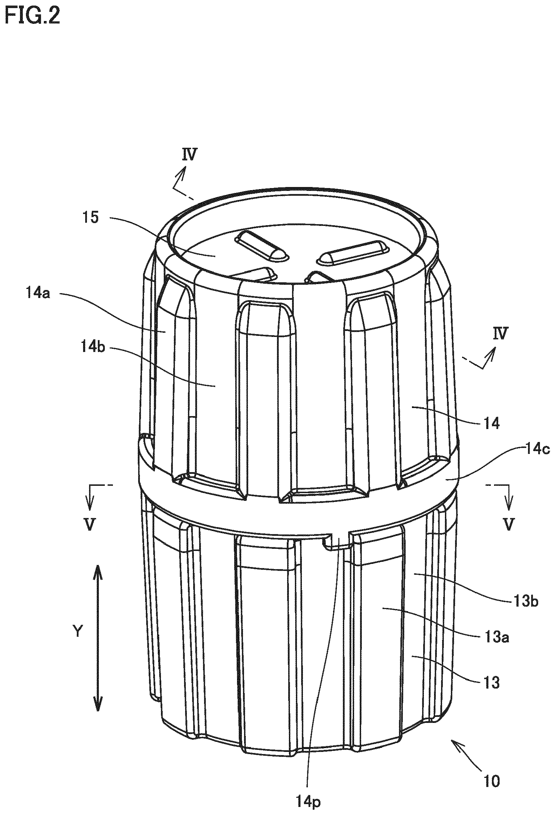

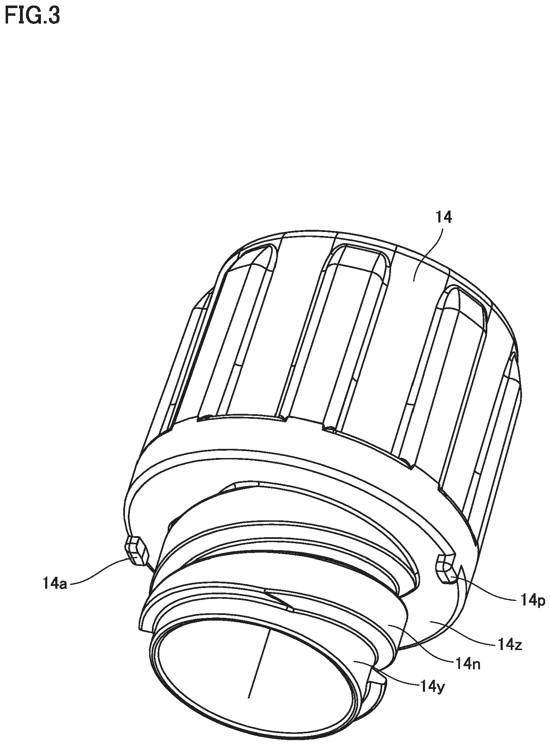

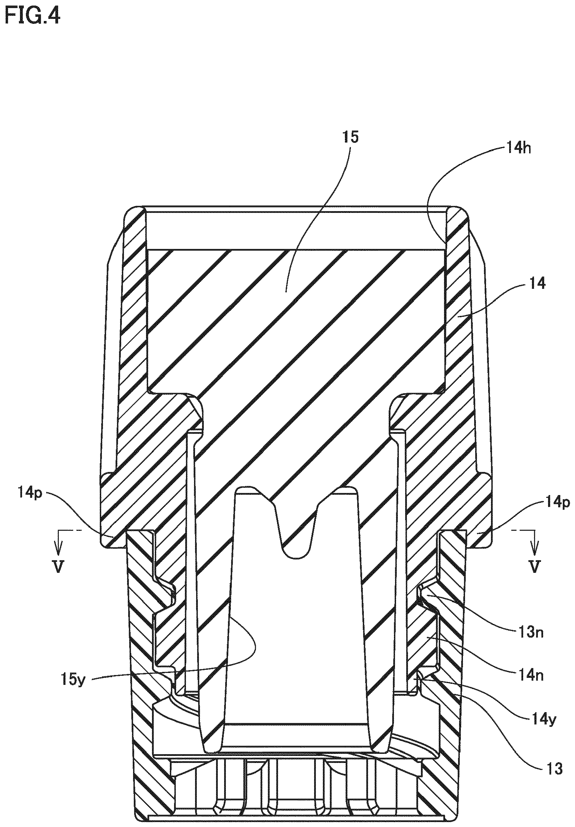

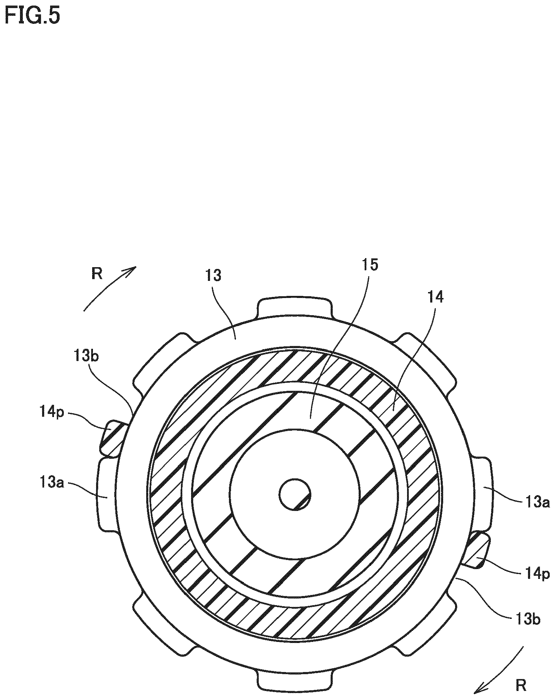

Referring to Figs. 1 to 5, a configuration of a prefilled syringe 1 according to an embodiment is described. Fig. 1 shows an overall configuration of prefilled syringe 1, Fig. 2 is a perspective view showing a syringe cap assembly 10 to be attached to the proximal end of a syringe 11, Fig. 3 is a perspective view as seen from the bottom of a plastic cap 14a, Fig. 4 is a cross-sectional view as seen in the direction of an arrow-headed line IV-IV in Fig. 2, and Fig. 5 is a perspective view as seen in the direction of an arrow-headed line V-V in Fig. 4.

Referring to Fig. 1, prefilled syringe 1 includes syringe 11 to be filled with a medical liquid or the like, a piston 12 to be inserted in syringe 11, and syringe cap assembly 10 attached to an ejection port at the proximal end of syringe 11.

Syringe cap assembly 10 includes a lock collar 13, a plastic cap 14 screwed into lock collar 13, and a rubber cap 15 inserted in plastic cap 14.

Prefilled syringe 1 usually means a condition where syringe 11 has been filled with a liquid medicine or the like. While Fig. 1 shows syringe 11 into which piston 12 has been pushed in to the proximal end of syringe 11 and thus syringe 11 is not filled with the medical liquid any more, the syringe in this state is also referred to herein as prefilled syringe.

Referring to Figs. 2 to 4, syringe cap assembly 10 according to the present embodiment includes lock collar 13, plastic cap 14 screwed into lock collar 13, and rubber cap 15 inserted in plastic cap 14, as described above.

For plastic cap 14, a resin material such as polyvinyl chloride, polyethylene, polypropylene, cyclic polyolefin, polystyrene, polyacetal, poly(4-methylpentene-1), polycarbonate, acrylic resin, acrylonitrile-butadiene-styrene copolymer, polyester such as polyethylene terephthalate or polyethylene naphthalate, butadiene-styrene copolymer, polyamide (e.g. nylon 6, nylon 6-6, nylon 6-10, nylon 12), ethylene-vinyl alcohol copolymer, polysulfone, polyallylsulfone, polyethersulfone, methacryl-styrene copolymer, polyarylates, or styrene-acrylonitrile copolymer, for example, is used.

For rubber cap 15, any of various rubber materials such as natural rubber, isoprene rubber, butadiene rubber, styrene-butadiene rubber, nitrile rubber, chloroprene rubber, butyl rubber, acrylic rubber, ethylene-propylene rubber, Hydrin rubber, urethane rubber, silicone rubber, and fluororubber, or any of various thermoplastic elastomers such as styrene-based, polyethylene-based, polyvinyl chloride-based, polyurethane-based, polyester-based, polyamide-based, polybutadiene-based, transpolyisoprene-based, fluororubber-based, and chlorinated polyethylene-based thermoplastic elastomers, or a relatively soft resin material such as polyethylene or vinyl chloride resin, or a combination thereof, for example, may be used.

The external surface of lock collar 13 in a cylindrical shape has a plurality of projecting ribs 13a that are each extended in the axial direction (the direction indicated by arrows Y in the drawing) and are arranged in the circumferential direction. This configuration defines a plurality of recessed grooves 13b that are each extended in the axial direction and located between projecting ribs 13a adjacent to each other in the circumferential direction, among projecting ribs 13a. While projecting ribs 13a in the present embodiment are arranged at eight respective locations at regular intervals on the external surface of lock collar 13, arrangement of projecting ribs 13a is not limited to this arrangement and the number of projecting ribs 13a and the intervals at which the ribs are arranged, for example, may be changed appropriately depending on the specifications.

Like lock collar 13, the external surface of plastic cap 14 in a cylindrical shape also has a plurality of projecting ribs 14a that are each extended in the axial direction (the direction indicated by arrows Y in the drawing) and are arranged in the circumferential direction. This configuration defines a plurality of recessed grooves 14b that are each extended in the axial direction and located between projecting ribs 14a adjacent to each other in the circumferential direction, among projecting ribs 14a. The bottom side (lock collar 13 side) of the external surface of plastic cap 14 has an annular rib 14c surrounding the entire circumference.

While projecting ribs 14a in the present embodiment are arranged at eight respective locations at regular intervals on the external surface of plastic cap 14, arrangement of projecting ribs 14a is not limited to this arrangement and the number of projecting ribs 14a and the intervals at which the ribs are arranged, for example, may be changed appropriately depending on the specifications.

Referring to Figs. 2 and 3, a bottom 14z of the external surface of plastic cap 14 has a protrusion 14p extending in the direction toward lock collar 13. In the present embodiment, protrusion 14p is placed at each of total two locations facing each other with an angle of 180 degrees between the two locations.

Referring to Fig. 4, the inner circumferential surface of cylindrical lock collar 13 has a helical thread 13n, and the outer circumferential surface of a bottom cylindrical portion 14y that extends from the bottom of cylindrical plastic cap 14 in the direction toward lock collar 13 has a thread 14n screwed with helical thread 13n. Rubber cap 15 mentioned above is fit into an inner circumferential surface 14h of plastic cap 14.

The bottom of rubber cap 15 has a recess 15y extending in the axial direction. In the state where plastic cap 14 has been screwed and tightened in lock collar 13, recess 15y is pressed liquid-tight against the ejection port at the proximal end of syringe 11.

Referring to Figs. 2 and 5, at the stage where tightening rotation (in the direction indicated by arrows R in Fig. 5) of plastic cap 14 that is implemented by screwing plastic cap 14 into lock collar 13 is completed, protrusion 14p may be located in recessed groove 13b between projecting ribs 13a. More preferably, as clearly illustrated in Fig. 5, protrusion 14p may abut against a sidewall of projecting rib 13a that is located upstream in the rotational direction of plastic cap 14.

Thus, in syringe cap assembly 10 according to the present embodiment, at the stage where tightening rotation (in the direction of arrows R in Fig. 5) of plastic cap 14 that is implemented by screwing plastic cap 14 into lock collar 13 is completed, protrusion 14p is located in recessed groove 13b between projecting ribs 13a. Accordingly, even if plastic cap 14 is loosened from lock collar 13, protrusion 14p abuts against the sidewall of projecting rib 13a to thereby enable prevention of rotation of plastic cap 14.

More preferably, a design can be made to allow the protrusion to abut against the sidewall of projecting rib 13a that is located upstream in the rotational direction of plastic cap 14, at the stage where tightening rotation (in the direction of arrows R in Fig. 5) implemented by screwing plastic cap 14 into lock collar 13 is completed, as clearly illustrated in Fig. 5, to prevent plastic cap 14 from rotating after tightening of plastic cap 14 implemented by screwing plastic cap 14 into lock collar 13 is completed. Plastic cap 14 may not necessarily be required to abut against the sidewall of projecting rib 13a, and plastic cap 14 located near this sidewall also produces a substantially equivalent advantageous effect.

While the foregoing embodiment employs the configuration where projecting ribs 14a are arranged at eight respective locations at regular intervals on the external surface of plastic cap 14 and protrusion 14p is placed at each of total two locations facing each other with an angle of 180 degrees between the two locations, its configuration is not limited to this configuration and protrusion 14p may be provided at one or more locations.

The above embodiments disclosed herein are given by way of illustration in all respects, not as grounds for limited interpretation. The technical scope of the present invention is therefore defined by the description of claims, not to be interpreted solely from the above embodiments, and encompasses all variations equivalent in meaning and scope to the claims.

### REFERENCE SIGNS LIST

1 prefilled syringe; 11 syringe; 12 piston; 10 syringe cap assembly; 13 lock collar; 13a projecting rib; 13b recessed groove; 13n helical thread; 14 plastic cap; 14a projecting rib; 14b recessed groove; 14c annular rib; 14h inner circumferential surface; 14n thread; 14p protrusion; 14y bottom cylindrical portion; 14z bottom; 15 rubber cap; 15y recess

## Claims

1. A syringe cap assembly to be attached to an ejection port at a proximal end of a syringe, the syringe cap assembly comprising:
a lock collar to be attached to the ejection port at the proximal end of the syringe; and
a plastic cap screwed and thereby tightened into the lock collar, wherein
an external surface of the lock collar has a plurality of projecting ribs that are each extended in an axial direction and are arranged in a circumferential direction to define a plurality of recessed grooves, each recessed groove of the plurality of recessed grooves is extended in the axial direction and located between adjacent projecting ribs of the plurality of projecting ribs, and the adjacent projecting ribs are adjacent to each other in the circumferential direction,
a bottom of an external surface of the plastic cap includes a protrusion extending in a direction toward the lock collar, and
at a stage where tightening rotation of the plastic cap implemented by screwing the plastic cap into the lock collar is completed, the protrusion is located in the recessed groove between the adjacent projecting ribs.

2. The syringe cap assembly according to claim 1, wherein
the plurality of projecting ribs are arranged at regular intervals on the external surface of the lock collar to allow the plurality of recessed grooves to be defined at regular intervals on the external surface of the lock collar, and
at the stage where tightening rotation of the plastic cap implemented by screwing the plastic cap into the lock collar is completed, the protrusion is located near one of the projecting ribs that is located upstream in a rotational direction of the plastic cap.

3. The syringe cap assembly according to claim 2, wherein the plurality of projecting ribs are arranged at eight respective locations at regular intervals on the external surface of the lock collar.

4. The syringe cap assembly according to any one of claims 1 to 3, wherein the protrusion is placed at each of total two locations facing each other with an angle of 180 degrees between the two locations.
